# EUROPEAN PATENT APPLICATION

(11) **EP 3 992 279 A1**
(43) Date of publication of application: **04.05.2022**
(21) Application number: 20772583.9
(22) Date of filing: 13.03.2020
(51) Int. Cl.: C12N 5/078, C12N 5/0783, C12N 9/12, C07K 14/52, C07K 14/55, A61K 35/17, A61K 47/64, A61K 47/65, A61P 35/00

(54) **CYTOKINE-BASED IMMUNE CELLS AND IMMUNOTHERAPEUTIC USE THEREOF**

(30) Priority: 15.03.2019 KR 20190030022
(71) Applicant: CTCELLS, Daegu 42988 (KR)
(72) Inventor: KIM, Min Seok, Dalseong-gun, Daegu 42988 (KR); YEA, Kyung Moo, Dalseong-gun, Daegu 43016 (KR); SHIN, Hyun Young, Suwon-si, Gyeonggi-do 16700 (KR)
(74) Representative: Ostertag & Partner Patentanwälte mbB
(86) International application number: PCT/KR2020/003523
(87) International publication number: WO 2020/189972

(57) **Abstract**

The present invention relates to cytokine-based immune cells and an immunotherapeutic use thereof, and an immune cell according to an aspect is designed in such a form that a cytokine is linked to the surface of the cell via a linker, and thus the cytokine continuously stimulates the immune cell, thereby inducing proliferation and activation, and the cytokine does not affect surrounding cells, whereby side effects can be minimized.

<110>    Daegu Gyeongbuk Institute of Science and Technology

<120>    Immune cells based on cytokines and immunotherapy uses thereof

<130>    PX061411PCT

<150>    KR 10-2019-0030022
<151>    2019-03-15

<160>    11

<170>    KoPatentIn 3.0

<210>    1
<211>    5
<212>    PRT
<213>    Artificial Sequence

<220>
<223>    Linker 1


<400>    1
Gly Gly Gly Gly Ser
  1               5


<210>    2
<211>    5
<212>    PRT
<213>    Artificial Sequence

<220>
<223>    Linker 2


<400>    2
Ser Gly Gly Gly Gly
  1               5


<210>    3
<211>    5
<212>    PRT
<213>    Artificial Sequence

<220>
<223>    Linker 3


<400>    3
Ser Arg Ser Ser Gly
  1               5


<210>    4
<211>    5
<212>    PRT
<213>    Artificial Sequence

<220>
<223>    Linker 4

<400>    4
Ser Gly Ser Ser Cys
1               5

<210>    5
<211>    12
<212>    PRT
<213>    Artificial Sequence

<220>
<223>    Linker 5

<400>    5
Gly Lys Ser Ser Gly Ser Gly Ser Glu Ser Lys Ser
1               5                   10

<210>    6
<211>    6
<212>    PRT
<213>    Artificial Sequence

<220>
<223>    Linker 6

<400>    6
Arg Pro Pro Pro Pro Cys
1               5

<210>    7
<211>    7
<212>    PRT
<213>    Artificial Sequence

<220>
<223>    Linker 7

<400>    7
Ser Ser Pro Pro Pro Pro Cys
1               5

<210>    8
<211>    14
<212>    PRT
<213>    Artificial Sequence

<220>
<223>    Linker 8

<400>    8
Gly Ser Thr Ser Gly Ser Gly Lys Ser Ser Glu Gly Lys Gly
1               5                   10

<210>    9

<211>    18
<212>    PRT
<213>    Artificial Sequence

<220>
<223>    Linker 9


<400>    9
Gly Ser Thr Ser Gly Ser Gly Lys Ser Ser Glu Gly Ser Gly Ser Thr
1               5                   10                  15

Lys Gly




<210>    10
<211>    18
<212>    PRT
<213>    Artificial Sequence

<220>
<223>    Linker 10


<400>    10
Gly Ser Thr Ser Gly Ser Gly Lys Pro Gly Ser Gly Glu Gly Ser Thr
1               5                   10                  15

Lys Gly




<210>    11
<211>    14
<212>    PRT
<213>    Artificial Sequence

<220>
<223>    Linker 11


<400>    11
Glu Gly Lys Ser Ser Gly Ser Gly Ser Glu Ser Lys Glu Phe
1               5                   10

## Description

### Technical Field

The present invention relates to cytokine-based immune cells and an immunotherapeutic use thereof.

### Background Art

Anti-cancer treatments using immune cells have very few problems of side effects such as systemic toxicity caused by existing first-generation anti-cancer therapeutic agents or the risk of recurrence of second-generation anti-cancer therapeutic agents, are superior compared to existing therapeutic agents in terms of excellent anti-cancer effects, and thus are a field that has received a lot of attention recently.

Among them, an immune cell treatment using NK cells is one of the anti-cancer treatment therapies known as the fourth anti-cancer treatment, and has an advantage of being the only cell treatment capable of using the immune cells of others without side effects. Among various immune cells, natural killer cells can be administered as a cell therapeutic agent even when the natural killer cells are not the patient's own cells unlike T cells, and accordingly, can be developed and used as a therapeutic agent at relatively low cost. Further, due to their high cytotoxicity, natural killer cells can be used not only for the treatment of hematologic cancer which has been confirmed to be effective using T cells, but also for the treatment of solid cancer, and thus have a great potential as a cell therapeutic agent for the treatment of more various types of cancers.

For the past 10 years or so, a tumor immune treatment using the immune system of patients has been continuously developed, and a cell therapeutic agent using the same has also been commercialized. Recently, a cell therapeutic agent, which treats cancer by isolating NK cells from the blood of a healthy normal person, culturing the NK cells, and injecting the culture NK cells into a patient with cancer, has been developed, and a large-scale culture process for the cell therapeutic agent, and the like have been actively studied. In particular, natural killer cells have been proliferated and activated using cytokines such as interleukin-2 in the related art. Since natural killer cells die when interleukin-2 is not present, , natural killer cells are proliferated and activated by introducing an interleukin-2 gene into natural killer cells to allow interleukin-2 to be secreted, or supplying interleukin-2 from the outside.

However, in such a method, a high concentration of interleukin-2 may induce an inflammatory reaction, and cells secreting interleukin-2 affect surrounding cells at a local site, such that a possibility of side effects is present.

Therefore, there is a need for the development of cytokine-based natural killer cells that prevent side effects while using cytokines for the proliferation and activation of natural killer cells.

### Disclosure

### Technical Problem

An aspect is to provide genetically engineered immune cells including: a transmembrane domain; a cytokine; and a peptide linker for linking the cytokine to the transmembrane domain.

Another aspect is to provide a cell therapeutic agent including the immune cells or a cell population thereof as an active ingredient.

Still another aspect is to provide a pharmaceutical composition for preventing or treating cancer, including the immune cells or a cell population thereof as an active ingredient.

Yet another aspect is to provide a pharmaceutical composition for preventing or treating an infectious disease, including the immune cells or a cell population thereof as an active ingredient.

Yet another aspect is to provide a method for preventing or treating a cancer or an infectious disease, the method including administering the immune cells or a cell population thereof to an individual in need thereof.

Yet another aspect is to provide a use of the immune cells or a cell population thereof for manufacture of a medicament for prevention or treatment a cancer or an infectious disease.

### Technical Solution

An aspect provides genetically engineered immune cells including: a transmembrane domain; a cytokine; and a peptide linker for linking the cytokine to the transmembrane domain.

As used herein, the term "transmembrane domain" refers to a region that penetrates a cell membrane in a protein that breaks through the cell membrane and is present, and most of the transmembrane domain is known to include hydrophobic amino acids having an α-helical structure. The transmembrane domain may serve to enable sustained stimulation by immobilizing a cytokine on the cell membrane or plasma membrane of a target cell and displaying a cytokine bound thereto on the surface of the target cell or binding a cytokine to a cell membrane receptor of the target cell. The transmembrane domain may be a transmembrane domain of receptor tyrosine kinases (RTKs). More specifically, the transmembrane domain of receptor tyrosine kinases may be a transmembrane domain of any one receptor selected from the group consisting of an epidermal growth factor receptor, an insulin receptor, a platelet-derived growth factor receptor, a vascular endothelial growth factor receptor, a fibroblast growth factor receptor, a cholecystokinin (CCK) receptor, a neurotrophic factor (NGF) receptor, a hepatocyte growth factor (HGF) receptor, an ephrin (Eph) receptor, an angiopoietin receptor, and a related to receptor tyrosine kinase (RTK) receptor.

As used herein, the term "cytokines" may refer to proteins (about 5 to 20 kDa) that play a role in signal transduction. Cytokines are released by cells, and affect the behavior of cells which release cytokines and/or other cells. Non-limiting examples of cytokines include chemokines, interferons, interleukins, lymphokines, tumor necrosis factors, monokines, and colony stimulating factors. Cytokines may be produced by a wide range of cells including (but not limited thereto) immune cells, for example, macrophages, B lymphocytes, T lymphocytes, mast cells and monocytes, endothelial cells, fibroblasts, and stromal cells. Cytokines may be produced by one or more types of cells. Cytokines act through receptors, and are especially important in the immune system, modulate the balance between humoral and cell-based immune responses, and regulate the maturation, growth and responsiveness of cell populations. The cytokine of the present specification may be a naturally occurring cytokine or a mutated version of the naturally occurring cytokine. As used herein, the "naturally occurring" may be also referred to as a wild type, and includes allelic variants. The mutated version of the naturally occurring cytokine or a "mutant" refers to particular mutations made to a naturally occurring sequence to alter the function, activity and/or specificity of the cytokine. In one specific example, mutations may improve the function, activity and/or specificity of the cytokine. In another specific example, mutations may reduce the function, activity and/or specificity of the cytokine. Mutations may include the deletion or addition of one or more amino acid residues of the cytokine.

The cytokine may be any one selected from the group consisting of the bone morphogenetic protein (BMP) family, the chemokine ligand (CCL) family, the CKLF-like MARVEL transmembrane domain-containing member (CMTM) family, the C-X-C motif ligand (CXCL) family, the growth/differentiation factor (GDF) family, growth hormones, the interferon (IFN) family, the interleukin (IL) family, the tumor necrosis factor superfamily (TNFSF), glycophosphatidylinositol (GPI), secreted *Ly-6*/*uPAR-*related protein 1 (SLUPR-1), secreted *Ly-6*/*uPAR-*related protein 2 (SLUPR-2) and a combination thereof.

In one specific example, the cytokine is an interleukin or a mutant thereof. A number of interleukins are synthesized by monocytes, macrophages, and endothelial cells, as well as auxiliary CD4T lymphocytes. Interleukins may promote the development and differentiation of T and B lymphocytes and hematopoietic cells. Non-limiting examples of interleukins include IL1, IL2, IL3, IL4, IL5, IL6, IL7, IL8 (CXCL8), IL9, IL10, IL11, IL12, IL13, IL14, IL15, IL16, IL17, IL18, IL19, IL20, IL21, IL22, IL23, IL24, IL25, IL26, IL27, IL28, IL29, IL30, IL31, IL32, IL33, IL35, or IL36. Therefore, in certain exemplary embodiments, the cytokine is an interleukin or a mutant thereof including (but not limited thereto) wild-type or mutated forms of IL1, IL2, IL3, IL4, IL5, IL6, IL7, IL8 (CXCL8), IL9, IL10, IL11, IL12, IL13, IL14, IL15, IL16, IL17, IL18, IL19, IL20, IL21, IL22, IL23, IL24, IL25, IL26, IL27, IL28, IL29, IL30, IL31, IL32, IL33, IL35, or IL36. In one certain specific example, the cytokine is IL2 of a mutant thereof. IL2 is a lymphokine that induces the proliferation of responsive T cells. Further, it acts on some B cells, via receptor-specific binding, as a growth factor and antibody production stimulant. An IL2 protein is secreted as a single glycosylated polypeptide, and cleavage of a signal sequence is required for its activity. The structure of IL-2 comprises a bundle of 4 helices (named A-D), flanked by two shorter helices and several poorly defined loops. Residues in helix A, and in the loop regions between helices A and B, are important for receptor binding. Secondary structure analysis has suggested similarities to IL4 and a granulocyte-macrophage colony stimulating factor (GMCSF). In one certain specific example, the cytokine of the present composition is IL2 or a variant thereof. The variant may be truncated or mutated IL2.

In another specific example, the cytokine is an interferon subfamily or a mutant thereof. Based on the receptor type where interferons transduce signals, human interferons have been classified into three main types: type I IFN, type II IFN, and type III IFN. Type I IFNs bind to a specific cell surface receptor complex known as an IFN-α/β receptor (IFNAR), which consists of IFNAR1 and IFNAR2 chains. Non-limiting examples of the type I interferon present in humans include IFN-α, IFN-β, IFN-ε, IFN-κ, and IFN-ω. Therefore, in certain exemplary embodiments, the cytokines is the type I IFN cytokine or a mutant thereof including (but not limited to) a wild type and mutant forms of IFN-α, IFN-β, IFN-ε, IFN-κ, and IFN-ω. Type II IFNs bind to IFNGR consisting of IFNGR1 and IFNGR2 chains. Non-limiting examples of the type II interferon present in humans include IFN-γ. Therefore, in certain exemplary embodiments, the cytokine is the type II IFN cytokine or a mutant thereof including (but not limited to) a wild type and a mutant form of IFN-γ. Type III IFNs transduce signals via a receptor complex consisting of IL10R2 (also termed CRF2-4) and IFNLR1 (also termed CRF2-12). Non-limiting examples of the type III interferon include IFN-λ1, IFN-λ2, and IFN-λ3 (also called IL29, IL28A, and IL28B, respectively). Therefore, in certain exemplary embodiments, the cytokine of the present composition is the type III IFN cytokine or a mutant thereof including (but not limited thereto) a wild type and mutant forms of IFN-λ1, IFN-λ2, and IFN-λ3.

In another specific example, the cytokine is a member of the tumor necrosis factor superfamily (TNFSF), or a mutant thereof. Members of the TNFSF induce inflammatory conditions and stimulate immune cell functions with pro-inflammatory cytokines, which are primarily expressed by immune cells. There are at least 18 TNFSF homologues including (but not limited thereto) TNF (TNF alpha), CD40L (TNFSF5), CD70 (TNFSF7; CD27L), EDA, FASL (TNFSF6; Fas ligand), LTA (TNFSF1; lymphotoxin-alpha), LTB (TNFSF3; lymphotoxin-beta), TNFSF4 (OX40L), TNFSF8 (CD153), TNFSF9 (4-1BBL), TNFSF10 (TRAIL), TNFSF11 (RANKL; receptor activator of NF-nuclear factor kappaB ligand), TNFSF12 (TWEAK), TNFSF13, TNFSF13B, TNFSF14, TNFSF15, TNFSF18. Therefore, in certain exemplary embodiments, the cytokine of the present composition is a member of the tumor necrosis factor superfamily (TNFSF) including TNF (TNF alpha), CD40L (TNFSF5), CD70 (TNFSF7; CD27L), EDA, FASL (TNFSF6), LTA (TNFSF1), LTB (TNFSF3), TNFSF4 (OX40L), TNFSF8 (CD153), TNFSF9 (4-1BBL), TNFSF10 (TRAIL), TNFSF11 (RANKL), TNFSF12 (TWEAK), TNFSF13, TNFSF13B, TNFSF14, TNFSF15, TNFSF18, or a mutant thereof (but is not limited thereto).

In another specific example, the CXCL may be CXCL1, CXCL 2, CXCL 5, CXCL 9, CXCL 10, CXCL 11,CXCL 12, CXCL 13, CXCL 14, CXCL 16, or CXCL 17.

In another specific example, the chemokine (c-c motif) ligand (CCL) may be CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL24, CCL25, CCL26, CCL27, or CCL28.

In another specific example, the BMP may be BMP1, BMP2, BMP3, BMP4, BMP5, BMP6, BMP7, BMP8, BMP9, BMP10, BMP11, BMP12, BMP13, BMP14, or BMP 15.

The linker may serve not only to link the cytokine to the transmembrane domain but also to expose the cytokine at the surface of a target cell depending on the flexibility of the linker. As the linker, for example, a flexible linker may be applied. In addition, the linker may have resistance to proteolytic enzymes (protease resistance), and the linker may be used by appropriately changing the type and/or length thereof depending on a cytokine or a target cell. For example, the linker may be a polypeptide consisting of any 1 to 400, 1 to 200, or 2 to 200 amino acids. The peptide linker may include Gly, Asn and Ser residues, and may also include neutral amino acids such as Thr and Ala. Amino acid sequences suitable for peptide linkers are known in the art. Furthermore, the copy number "n" may be adjusted in consideration of the optimization of a linker to achieve appropriate separation between functional parts or to maintain an essential inter-moiety interaction. Other flexible linkers are known in the art, and for example, there may be G and S linkers with amino acid residues such as T and A added to maintain flexibility as well as polar amino acid residues added to improve water solubility. Therefore, in an exemplary embodiment, the linker may be a flexible linker including G, S, and/or T residues. The linker may have a general formula selected from (GₚSₛ)ₙ and (SₚGₛ)ₙ, and in this case, independently, p is an integer from 1 to 10, s is 0 or an integer of 0 to 10, p + s is an integer of 20 or less, and n is an integer of 1 to 20. More specifically, examples of the linker include (GGGGS)n (SEQ ID NO: 1), (SGGGG)n (SEQ ID NO: 2), (SRSSG)n (SEQ ID NO: 3), (SGSSC)n (SEQ ID NO: 4), (GKSSGSGSESKS)n (SEQ ID NO: 5), (RPPPPC)n (SEQ ID NO: 6), (SSPPPPC)n (SEQ ID NO: 7), (GSTSGSGKSSEGKG)n (SEQ ID NO: 8), (GSTSGSGKSSEGSGSTKG)n (SEQ ID NO: 9), (GSTSGSGKPGSGEGSTKG)n (SEQ ID NO: 10), or (EGKSSGSGSESKEF)ₙ (SEQ ID NO: 11), and n is an integer from 1 to 20, or from 1 to 10.

In a specific example, non-limiting examples of the immune cells include macrophages, B lymphocytes, T lymphocytes, mast cells, monocytes, dendritic cells, eosinophils, natural killer cells, basophils, and neutrophils. Therefore, in certain exemplary embodiments, the immune cells may be any one selected from the group consisting of macrophages, B lymphocytes, T lymphocytes (CD8+ CTL), mast cells, monocytes, dendritic cells, eosinophils, natural killer cells, basophils, and neutrophils. In certain specific examples, the immune cells may be natural killer cells or T lymphocytes.

As used herein, the term "natural Killer cells" or "NK cells" is defined as a large granular lymphocyte (LGL) as cytotoxic lymphocytes that make up the major components of the innate immune system, and produces a common lymphoid progenitor (CLP) and constitutes a third cell differentiated from B and T lymphocytes. The "natural killer cells" or "NK cells" includes natural killer cells without additional modification derived from any tissue source, and may include not only mature natural killer cells but also natural killer progenitor cells. The natural killer cells are activated by a response to interferon or macrophage-derived cytokines, and the natural killer cells include two types of surface receptors, which are labeled with "activating receptors" and "inhibitory receptors" and control the cytotoxic activity of the cells. Natural killer cells may be produced from any source, for example, hematopoietic cells from placental tissue, placental perfusate, cord blood, placental blood, peripheral blood, the spleen, the liver, and the like, for example, hematopoietic stem and progenitor cells.

In a specific example, the natural killer cell may be an activated natural killer cell. The activated natural killer cell may mean a cell in which cytotoxicity or the original immunomodulatory ability of the natural killer cell is activated compared to a parent cell, for example, a hematopoietic cell or a natural killer progenitor cell. In specific exemplary embodiments, the activated natural killer cell is CD3-CD56+. In specific exemplary embodiments, the activated natural killer cell is CD3-CD56+CD16-. In other specific exemplary embodiments, the activated natural killer cell is additionally CD94+CD117+. In other specific exemplary embodiments, the activated natural killer cell is additionally CD161-. In other specific exemplary embodiments, the activated natural killer cell is additionally NKG2D+. In other specific exemplary embodiments, the activated natural killer cell is additionally NKp46+. In other specific exemplary embodiments, the activated natural killer cell is additionally CD226+. In certain exemplary embodiments, more than 50%, 60%, 70%, 80%, 90%, 92%, 94%, 96%, or 98% of the activated natural killer cells are CD56+ and CD16-. In other exemplary embodiments, at least 50%, 60%, 70%, 80%, 82%, 84%, 86%, 88% or 90% of the activated natural killer cells are CD3- and CD56+. In other exemplary embodiments, at least 50%, 52%, 54%, 56%, 58% or 60% of the activated natural killer cells are NKG2D+. In other exemplary embodiments, 30%, 20%, 10%, 9%, 8%, 7%, 6%, 5%, 4% or 3% of the activated natural killer cells are NKB1+. In certain other exemplary embodiments, less than 30%, 20%, 10%, 8%, 6%, 4% or 2% of the activated natural killer cells are NKAT2+. In certain other certain exemplary embodiments, less than 30%, 20%, 10%, 8%, 6%, 4% or 2% of the activated natural killer cells are CD56+ and CD16+. In more specific exemplary embodiments, at least 10%, 20%, 25%, 30%, 35%, 40%, 50%, 55%, 60%, 65% or 70% of the CD3-, CD56+ activated natural killer cells are NKp46+. In other more specific exemplary embodiments, at least 10%, 20%, 25%, 30%, 35%, 40%, 50%, 55%, 60%, 65%, 70%, 75%, 80% or 85% of the CD3-, CD56+ activated natural killer cells are CD117+. In other more specific exemplary embodiments, at least 10%, 20%, 25%, 30%, 35%, 40%, 45% or 50% of the CD3-, CD56+ activated natural killer cells are CD94+. In other more specific exemplary embodiments, at least 10%, 20%, 25%, 30%, 35%, 40%, 45% or 50% of CD3-, CD56+ activated natural killer cells are CD161-. In other more specific exemplary embodiments, at least 10%, 12%, 14%, 16%, 18% or 20% of the CD3-, CD56+ activated natural killer cells are CD226+. In other more specific exemplary embodiments, at least 20%, 25%, 30%, 35% or 40% of the CD3-, CD56+ activated natural killer cells are CD7+. In other more specific exemplary embodiments, at least 30%, 35%, 40%, 45%, 50%, 55% or 60% of the CD3-, CD56+ activated natural killer cells are CD5+.

In a specific example, the activated natural killer cells or activated natural killer cell-enriched populations may be assessed by detecting one or more functionally relevant markers, for example, CD94, CD161, NKp44, DNAM-1, 2B4, NKp46, CD94, KIR, and the NKG2 family of activating receptors (for example, NKG2D).

In a specific example, the activated natural killer cells may be produced by the above-described hematopoietic cells. In certain exemplary embodiments, the activated natural killer cells may be obtained from proliferated hematopoietic cells, for example, hematopoietic stem cells and/or hematopoietic progenitor cells. In specific exemplary embodiments, the hematopoietic cells proliferate and differentiate continuously in a first medium without using feeder cells. Thereafter, the cells are cultured in a second medium in the presence of feeder cells. Such separation (isolation), proliferation and differentiation may be performed in a central facility, which provides proliferated hematopoietic cells for proliferation and differentiation at a point of use, such as in a hospital, and the like.

In a specific example, the production of activated natural killer cells includes proliferating a population of hematopoietic cells. During cell proliferation, a plurality of hematopoietic cells within a hematopoietic cell population differentiate into natural killer cells.

As used herein, the term "natural killer progenitor cells", or "NK progenitor cells", or a cell population thereof may refer to cells or a population thereof including cells of a natural killer cell lineage, which have not yet developed into mature natural killer cells, for example, as indicated by the expression levels of one or more phenotypic markers, such as CD56, CD16, and KIR. In an exemplary embodiment, a natural killer progenitor cell population includes cells having low CD16 and high CD56. For example, a natural killer progenitor cell population includes about 5%, 10%, 15%, 20%, 25%, 30%, 35%, 40%, 45%, or 50% CD3-CD56+ cells. In other specific exemplary embodiments, the natural killer progenitor cell population includes 5%, 10%, 15%, 20%, 25%, 30%, 35%, 40%, 45%, or 50% CD3-CD56+ cells or less. In other specific exemplary embodiments, the natural killer progenitor cell population includes 0% to 5%, 5% to 10%, 10% to 15%, 15% to 20%, 20% to 25%, 25% to 30%, 30% to 35%, 35% to 40%, 40% to 45%, or 45% to 50% CD3-CD56+ cells.

In a specific example, in the natural killer progenitor cell population, the CD3-CD56+ cell is additionally CD117+. In specific exemplary embodiments, in the natural killer progenitor cell population, about 65%, 70%, 75%, 80%, 85%, 90%, 95%, 98%, or 99% of the CD3-CD56+ cells are CD117+. In other specific exemplary embodiments, in the natural killer progenitor cell population, 65%, 70%, 75%, 80%, 85%, 90%, 95%, 98%, or 99% or more of the CD3-CD56+ cells are CD117+. In other specific exemplary embodiments, in the natural killer progenitor cell population, 65% to 70%, 70% to 75%, 75% to 80%, 80% to 85%, 85% to 90%, 90% to 95%, or 95% to 99% of the CD3-CD56+ cells are CD117+.

In another specific example, in the natural killer progenitor cell population, the CD3-CD56+ cell is additionally CD161+. In specific exemplary embodiments, in the natural killer progenitor cell population, about 40%, 45%, 50%, 55%, 60%, 65%, 70%, or 75% of the CD3-CD56+ cells are CD161+. In other specific exemplary embodiments, in the natural killer progenitor cell population, 40%, 45%, 50%, 55%, 60%, 65%, 70%, or 75% or more of the CD3-CD56+ cells are CD161+. In other specific exemplary embodiments, in the natural killer progenitor cell population, 40% to 45%, 45% to 50%, 50% to 55%, 55% to 60%, 60% to 65%, 65% to 70%, or 70% to 75% of the CD3-CD56+ cells are CD161+.

In still another specific example, in the natural killer progenitor cell population, the CD3-CD56+ cell is additionally NKp46+. In specific exemplary embodiments, in the natural killer progenitor cell population, about 25%, 30%, 35%, 40%, 45%, 50%, 55%, 60%, 65%, 70%, 75%, 80%, 85%, 90% or more of the CD3-CD56+ cells are NKp46+. In other specific exemplary embodiments, in the natural killer progenitor cell population, about 25%, 30%, 35%, 40%, 45%, 50%, or 55% of the CD3-CD56+ cells are NKp46+. In other specific exemplary embodiments, in the natural killer progenitor cell population, 25%, 30%, 35%, 40%, 45%, 50%, or 55% or less of the CD3-CD56+ cells are NKp46+. In other specific exemplary embodiments, in the natural killer progenitor cell population, 25% to 30%, 30% to 35%, 35% to 40%, 40% to 45%, 45% to 50%, 50% to 55%, 55% to 60%, 60% to 65%, 65% to 70%, 70% to 75%, 75% to 80%, 80% to 85%, 85% to 90% or more of the CD3-CD56+ cells are NKp46+. In more specific exemplary embodiments, in the natural killer progenitor cell population, 25% to 30%, 30% to 35%, 35% to 40%, 40% to 45%, 45% to 50%, or 50% to 55% of the CD3-CD56+ cells are NKp46+.

Further, for example, the natural killer progenitor cell population is as described above for CD52+, CD16+, CD244+ CD94+, or CD94+.

In the present specification, the immune cells (for example, natural killer cells) may be genetically modified or engineered.

As used herein, "genetic modification" or "genetic engineering" includes artificially altering the composition or structure of a genetic material in a cell.

In a specific example, immune cells (for example, natural killer cells) may be genetically modified to improve target specificity and/or homing specificity.

In a specific example, genetically modified immune cells (for example, natural killer cells) are natural killer cells including a chimeric antigen receptor (CAR). The CAR is an artificial membrane-binding protein that induces immune cells (for example, T lymphocytes) against an antigen, and kills cells showing the antigen by stimulating the immune cells. The CAR includes an extracellular domain which binds to an antigen, for example, an antigen on a cell, a transmembrane domain, and an intracellular (cytoplasmic) signaling domain (that is, an intracellular stimulatory domain) which transmits primary activation signals to immune cells and/or a co-stimulatory domain. When all other conditions are satisfied, the CAR is expressed on the surface of, for example, T lymphocytes, such as primary T lymphocytes, and an extracellular domain of the CAR binds to an antigen, the extracellular signaling domain activates and/or proliferates T lymphocytes by transmitting signals to the T lymphocytes, and kills cells expressing the antigen when the antigen is present on the cell surface. Some immune cells, for example, T lymphocytes and natural killer cells, require two signals for maximal activation, that is, a primary activation signal and a co-stimulatory signal, and the CAR may also include a co-stimulatory domain, such that a binding to the extracellular domain causes transmission of both the primary activation signal and the co-stimulatory signal.

In another specific example, genetically modified immune cells (for example, natural killer cells) may be immune cells (for example, natural killer cells) including a homing receptor. This allows the cells including the homing receptor to stay in a particular anatomical zone, particularly a tissue or particular type of cell, for example, a B cell zone of the lymph node, the gastrointestinal tract, or the skin.

In still another specific example, genetically modified immune cells (for example, natural killer cells) are immune cells including both the CAR and the homing receptor, as described herein.

Immune cells (for example, natural killer cells) including the CAR and/or the homing receptor may be produced by any method known in the art. In some exemplary embodiments, immune cells (for example, natural killer cells) including the CAR and/or the homing receptor are manipulated so as to express the CAR and/or the homing receptor by introducing (for example, by transfection) immune cells (for example, natural killer cells) into one or more vectors including nucleic acid sequence(s) encoding the CAR and/or the homing receptor. In some exemplary embodiments, cells (for example, CD34+ hematopoietic stem cells) in which immune cells (for example, natural killer cells) may be produced may be manipulated so as to express the CAR and/or the homing receptor by first introducing (for example, by transfection) one or more vectors including nucleic acid sequence(s) encoding the CAR and/or the homing receptor, and thereafter, immune cells (for example, natural killer cells) including the CAR and/or homing receptor may be induced by the method as described above.

In a specific example, an extracellular domain of the CAR is an antigen-binding domain. In specific exemplary embodiments, the antigen-binding domain is an scFv domain. In certain exemplary embodiments, the antigen-binding domain specifically binds to TAA. In specific exemplary embodiment, the TAA is selected from the group consisting of CD123, CLL-1, CD38, CD20, and CS-1. In more specific exemplary embodiments, the antigen-binding domain includes a single chain Fv (scFv) or antigen binding fragment derived from an antibody that binds to CS-1. In more specific exemplary embodiments, the antigen-binding domain includes a single chain of elotuzumab and/or an antigen-binding fragment of elotuzumab. In specific exemplary embodiments, the antigen-binding domain includes a single chain Fv (scFv) or antigen binding fragment derived from an antibody that binds to CD20.

In a specific example, an intracellular stimulatory domain of the CAR is a CD3 zeta signaling domain.

In a specific example, a co-stimulatory domain of the CAR includes an intracellular domain of CD28, 4-1BB, PD-1, OX40, CTLA-4, NKp46, NKp44, NKp30, DAP10 or DAP12.

In a specific example, the homing receptor is a chemotactic receptor. In specific exemplary embodiments, the chemotactic receptor is selected from the group consisting of CXCR4, VEGFR2, and CCR7.

In a certain specific example, the natural killer cell may be an NK-92 cell.

In a specific example, the cytokine may bind to a cytokine receptor present on the surface of the immune cell to continuously stimulate the cell.

As used herein, the term "stimulation of immune cells (for example, natural killer cells)" may mean that the activity, for example, the cytotoxic activity of immune cells (for example, natural killer cells) *in vitro* or *in vivo* is increased, or activated immune cells (for example, natural killer cells) are produced, increased, or proliferated.

According to the immune cells (for example, natural killer cells) according to a specific example, cytokines do not affect surrounding cells, so that proliferation and activation can be induced while minimizing side effects.

In a specific example, the immune cells may be transformed with a vector including a polynucleotide encoding a fusion protein including: a transmembrane domain; a cytokine; and a peptide linker for linking the cytokine to the transmembrane domain. Specifically, the immune cells may be immune cells including the vector. Therefore, as used herein, the term "genetically engineered immune cells" may refer to immune cells including those transformed with the vector.

The term "polynucleotide" refers to a polymer of deoxyribonucleotides or ribonucleotides present in the form of a single or double strand. The polynucleotide includes an RNA genome sequence, DNA (gDNA and cDNA) and an RNA sequence transcribed therefrom, and includes not only natural polynucleotides, but also their analogues with modified sugar or base sites, unless specifically stated otherwise. In a specific example, the polynucleotide is a single-chain polynucleotide.

As used herein, the term "vector" refers to a gene construct including a regulatory element that is operably linked to express a gene insert as a vector capable of expressing a target protein in a suitable host cell. A vector according to an example may include an expression regulatory element such as a promoter, an operator, a start codon, a stop codon, a polyadenylation signal, and/or an enhancer, and the promoter of the vector may be constitutive or inducible. Furthermore, the vector may be an expression vector capable of stably expressing the fusion protein in a host cell. As the expression vector, a typical vector used to express a foreign protein in a plant, animal or microorganism in the art may be used. The recombinant vector may be constructed by various methods known in the art. For example, the vector may include a selective marker for selecting a host cell containing the vector, and in the case of a replicable vector, may include a replication origin. Further, the vector may be self-replicating or introduced into host DNA, and the vector may be selected from the group consisting of a plasmid, lentivirus, adenovirus, adeno-associated virus, retrovirus, herpes simplex virus, and vaccinia virus.

The vector includes a promoter operable in animal cells, for example, mammalian cells. An appropriate promoter according to an example includes promoters derived from mammalian viruses and promoters derived from genomes of mammalian cells, and may include, for example, a cytomegalovirus (CMV) promoter, a U6 promoter and an H1 promoter, a long terminal repeat (LTR) promoter of murine leukemia virus (MLV), an adenovirus early promoter, an adenovirus late promoter, a vaccinia virus 7.5K promoter, a SV40 promoter, a tk promoter of HSV, an RSV promoter, an EF1 alpha promoter, a metallothionein promoter, a beta-actin promoter, a promoter of the human IL-2 gene, a promoter of the human IFN gene, a promoter of the human IL-4 gene, a promoter of the human lymphotoxin gene, a promoter of the human GM-CSF gene, a human phosphoglycerate kinase (PGK) promoter, a mouse phosphoglycerate kinase (PGK) promoter, and a survivin promoter.

In addition, in the vector, a polynucleotide sequence encoding the above-described fusion protein may be operably linked to the promoter. As used herein, the term "operably linked" refers to functional linkage between a nucleic acid expression regulatory sequence (for example: an array of a promoter, a signal sequence, and a transcription regulating factor-binding site) and a different nucleic acid sequence, and accordingly, the regulatory sequence regulates the transcription and/or translation of the different nucleic acid sequence.

Another aspect provides a cell therapeutic agent including the immune cells or a cell population thereof as an active ingredient.

Still another aspect provides a pharmaceutical composition for preventing or treating cancer or an infectious disease, including the immune cells or a cell population thereof as an active ingredient.

Yet another aspect provides a use of the immune cells or a cell population thereof for the manufacture of a medicament.

Yet another aspect provides a method for prevention or treatment a disease, the method including administering the immune cells or a cell population thereof to an individual.

As used herein, the term "disease" may refer to one pathological condition, particularly cancer, an infectious disease, an inflammatory disease, a metabolic disease, an autoimmune disorder, a degenerative disease, an apoptosis-related disease and graft rejection.

As used herein, the term "treatment" refers to the alleviation, progression suppression or prevention of a disease, disorder or condition, or one or more symptoms thereof, and includes the same, and "active ingredient" or "pharmaceutically effective amount" may mean any amount of composition used in the process of practicing the invention provided herein sufficient to alleviate, suppress the progression of or prevent the disease, disorder or condition, or one or more symptoms thereof.

As used herein, the terms "administering" "introducing" and "transplanting" are used interchangeably, and may refer to the disposition of a composition according to a specific example within an individual by a method or route for incurring at least partial localization of the composition according to a specific example at a desired site. At least some of the cells or cellular components of the composition according to a specific example may be administered by any suitable route which delivers the composition to a desired location within a living individual. The survival time of cells after administration to an individual may be as short as several hours, for example, 24 hours to several days or as long as several years.

As used herein, the term "isolated cells", such as "isolated immune cells" refers to cells which are substantially isolated from a tissue from which the cells originate, for example, hematopoietic cells.

The composition of the present invention may be used in methods of treating or recognizing a tumor or cancer derived from a neoplasm. The neoplasm may be malignant or benign, the cancer can be primary or metastatic, and the neoplasm or cancer may be early or late. Non-limiting examples of the neoplasm or cancer, which can be treated, include acute lymphoblastic leukemia, acute myelogenous leukemia, adrenocortical carcinoma, AIDS-related cancer, AIDS-related lymphoma, anal cancer, appendix cancer, astrocytoma (pediatric cerebellum or cerebrum)), basal cell carcinoma, cholangiocarcinoma, bladder cancer, bone tumors, brain stem glioma, brain tumors (cerebellar astrocytoma, cerebral astrocytoma/malignant glioma, ependymal glioma, medulloblastoma, supratentorial primitive neuroectodermal tumors, visual pathway and hypothalamic glioma), breast cancer, bronchial adenoma/carcinoid, Burkitt lymphoma, carcinoid tumors (pediatric, gastrointestinal), unknown primary carcinomas, central nervous system lymphoma (primary), cerebellar astrocytoma, cerebral astrocytoma/malignant glioma, cervical cancer, pediatric cancer, chronic lymphocytic leukemia, chronic myelogenous leukemia, chronic myeloproliferative disorders, colon cancer, cutaneous T-cell lymphoma, desmopolastic small round cell tumors, endometrial cancer, ependymal glioma, esophageal cancer, Ewing's sarcoma of the Ewing tumor family, extracranial germ cell tumors (pediatric), extragonadal germ cell tumors, extrahepatic bile duct cancer, eye cancer (intraocular melanoma, retinoblastoma), gallbladder cancer, gastric (stomach) cancer, gastrointestinal carcinoid tumors, gastrointestinal stromal tumors, germ cell tumors (pediatric extracranial, extragonadal, ovary), gestational trophoblastic neoplasia, glioma (adult, pediatric cerebral pediatric cerebral astrocytoma, pediatric visual pathway and hypothalamic), gastric carcinoid, hairy cell leukemia, head and neck cancer, hepatocellular (liver) cancer, Hodgkin lymphoma, hypopharyngeal cancer, hypothalamic and visual pathway glioma (pediatric), intraocular melanoma, islet cell carcinoma, Kaposi's sarcoma, renal cancer (renal cell carcinoma), laryngeal cancer, leukemia (acute lymphoblastic, acute myelogenous, chronic lymphocytic, chronic myelogenous, hairy cells), lip and oral cancer, liver cancer (primary), lung cancer (non-small cell, small cell), lymphoma (AIDS-related, Burkitt, cutaneous T-cell, Hodgkin, non-Hodgkin, primary central nervous system), macroglobulinemia (Waldenstrom), bone malignant fibrous histiocytoma/osteosarcoma, medulloblastoma (pediatric), melanoma, intraocular melanoma, Merkel cell carcinoma, mesothelioma (adult malignant, pediatric), metastatic squamous head and neck cancer with occult primary, oral cancer, multiple endocrine neoplasia syndrome (pediatric), multiple myeloma/plasma cell neoplasms, mycosis fungoides, myelodysplastic syndrome, myelodysplastic/myeloproliferative disorder, myelogenous leukemia (chronic), myelogenous leukemia (adult acute, pediatric acute), multiple myeloma, myeloproliferative disorders (chronic), nasal cavity and paranasal sinus cancer, nasopharyngeal carcinoma, neuroblastoma, non-Hodgkin lymphoma, non-small cell lung cancer, oral cancer, oropharyngeal cancer, osteosarcoma/malignant fibrous histiocytoma of bone, ovarian cancer, ovarian epithelial cancer (surface epithelial-stromal tumor), ovarian germ cell tumors, ovarian low malignant potential tumors, pancreatic cancer, pancreatic cancer (islet cell), paranasal sinus and nasal cavity cancer, parathyroid cancer, penile cancer, pharyngeal cancer, pheochromocytoma, pineal astrocytoma, pineal germinoma, pineoblastoma and supratentorial primitive neuroectodermal tumor (pediatric), pituitary adenoma, plasmacytic neoplasms, pleuropulmonary blastoma, primary central nervous system lymphoma, prostate cancer, rectal cancer, renal cell carcinoma (renal cancer), renal pelvis and ureter transitional cell cancer, retinoblastoma, rhabdomyosarcoma (pediatric), salivary gland cancer, sarcoma (Ewing family tumor, Kaposi, soft tissue, uterine tube), Sezary syndrome, skin cancer (non-melanoma, melanoma), skin carcinoma (Merkel cell), small cell lung cancer, small intestine cancer, soft tissue sarcoma, squamous cell carcinoma, (metastatic) squamous head and neck cancer with occult primary, gastric cancer, supratentorial primitive neuroectodermal tumors (pediatric), T-cell lymphoma (skin), testicular cancer, laryngopharyngeal cancer, thymoma (pediatric), thymoma and thymic carcinoma, thyroid cancer, thyroid cancer (pediatric), transitional cell cancer of the renal pelvis and ureter, trophoblastic neoplasia (gestational), unknown primary site (adult, pediatric), ureter and renal pelvic transitional cell cancer, urethral cancer, fallopian tube cancer (endometrium), fallopian tube sarcomas, vaginal cancer, visual pathway and hypothalamic glioma (pediatric), vulvar cancer, Waldenstrom macroglobulinemia, and Wilms' tumors (pediatric). In certain specific examples, the neoplasm or cancer may be selected from the group consisting of melanoma, renal cell carcinoma, lung cancer and hematologic cancer. In one specific example, the tumor is melanoma. In another specific example, the tumor is renal cell carcinoma. In one specific example, the tumor is lung cancer (for example, NSCLC). In still another specific example, the tumor is the hematologic cancer described in the present application. The "hematologic cancer" used in the present application is a cancer that affects the blood, bone marrow and lymphatic system. There are three major groups of hematologic cancer: leukemia, lymphoma and myeloma. The four broad categories of leukemia are as follows: acute lymphocytic leukemia (ALL), acute myelogenous leukemia (AML), chronic lymphocytic leukemia (CLL) and chronic myelogenous leukemia (CML). Lymphoma may be divided into two categories: Hodgkin lymphoma and non-Hodgkin lymphoma. Most non-Hodgkin lymphomas are B-cell lymphomas that grow rapidly (high-grade) or gradually (low-grade). There are 14 types of B-cell non-Hodgkin lymphoma. The others are different cancer white blood cells, or T-cell lymphoma named after lymphocytes. Myeloma is often referred to as multiple myeloma because it occurs frequently in many sites of the bone marrow. In some specific examples, these methods include the administration of combination therapy provided in the present application. In some specific examples, the combination therapy includes a PD-1 inhibitor. In other specific examples, the PD-1 inhibitor is an anti-PD-1 antibody. In some specific examples, the combination therapy includes a PD-L1 inhibitor. In other specific examples, the PD-L1 inhibitor is an anti-PD-Ll antibody.

As used herein, the term "infectious disease" includes the presence of a pathogen within or on a subject, wherein the subject is benefited when the growth of the pathogen is suppressed. Besides referring to the presence of a pathogen, such term "infection" also refers to an unfavorable group of normal bacteria. As used herein, the term "pathogen" refers to an infectious material capable of causing a disease. Non-limiting examples of the infectious material include viruses, bacteria, prions, fungi, viroids, or parasites that induce a disease in a subject. In one certain specific example, the infection is caused by pathogens, such as bacteria or viruses. In certain specific examples, the infection is an intracellular infection. In one specific example, the infection is a viral infection.

In a specific example, the method of administering a pharmaceutical composition is not particularly limited, but the pharmaceutical composition may be administered parenterally as in intravenous, subcutaneous, intraperitoneal, inhalation or topical application or orally depending on the desired method. A dosage varies according to the body weight, age, gender, and health status of a patient, diet, administration time, administration method, excretion rate, and the severity of a disease. A daily dose means the amount of therapeutic material according to one aspect sufficient to treat a disease condition that can be alleviated by administration to an individual in need of treatment. The effective amount of therapeutic material varies depending on the particular compound, the disease condition and severity thereof, and the individual in need of treatment, which may be typically determined by a person with ordinary skill in the art. As a non-limiting example, the dosage of the composition according to an aspect for the human body may vary depending on the age, body weight, and gender of a patient, mode of administration, health condition and degree of illness. Based on an adult patient having a body weight of 70 kg, the composition according to an aspect may be administered once or in several divided doses a day at regular time intervals, for example, about 1,000 to 10,000 cells/time, 1,000 to 100,000 cells/time, 1,000 to 1000,000 cells/time, 1,000 to 10,000,000 cells/time, 1,000 to 100,000,000 cells/time, 1,000 to 1,000,000,000 cells/time, and 1,000 to 10,000,000,000 cells/time, and may be administered several times at the regular time intervals.

The 'individual' refers to a subject in need of treatment of a disease, and more specifically, refers to a mammal such as a human or a non-human primate, a mouse, a rat, a dog, a cat, a horse, and a cow.

The pharmaceutical composition according to a specific example may include a pharmaceutically acceptable carrier and/or an additive. For example, the pharmaceutical composition may include sterilized water, saline, a commonly used buffer (such as phosphoric acid, citric acid, and other organic acids), a stabilizer, a salt, an antioxidant (such as ascorbic acid), a surfactant, a suspending agent, an isotonic agent, a preservative, or the like. For topical administration, the pharmaceutical composition may be combined with an organic material such as a biopolymer, an inorganic material such as hydroxyapatite, specifically, a combination of a collagen matrix, a polylactic acid polymer or copolymer, a polyethylene glycol polymer or copolymer, and chemical derivatives thereof, and the like. When the pharmaceutical composition according to a specific example is prepared in a dosage form suitable for injection, immune cells, or materials that increase their activity may be dissolved in a pharmaceutically acceptable carrier or may be frozen in the state of a dissolved solution.

The pharmaceutical composition according to a specific example may appropriately include a suspending agent, a solubilizing agent, a stabilizer, an isotonic agent, a preservative, an adsorption blocking agent, a surfactant, a diluent, an excipient, a pH adjusting agent, a pain reliever, a buffer, a reductant, an antioxidant, and the like, if necessary, according to the administration method or dosage form. Pharmaceutically acceptable carriers and formulations suitable for the present invention, including those exemplified above, are described in detail in the literature [Remington's Pharmaceutical Sciences, 19th ed., 1995]. The pharmaceutical composition according to a specific example may be prepared in the form of a unit-dose or by being contained in a multi-dose container by being formulated using a pharmaceutically acceptable carrier and/or excipient according to a method that can be readily implemented by a person with ordinary skill in the art to which the present invention pertains. In this case, the dosage form may be in the form of a solution, suspension or emulsion in an oil or aqueous medium, or in the form of powder, granules, tablets or capsules.

### Advantageous Effects

An immune cell according to an aspect is designed in such a form that a cytokine is linked to the surface of the cell via a linker, and thus the cytokine continuously stimulates the immune cell, thereby inducing proliferation and activation, and the cytokine does not affect surrounding cells, whereby side effects can be minimized.

### Description of Drawings

FIG. 1 is a schematic view of a natural killer cell according to an exemplary embodiment.
FIG. 2 is a cleavage diagram of a lentiviral vector for the production of natural killer cells according to an exemplary embodiment.
FIG. 3 is a graph showing the proliferative ability of natural killer cells according to an exemplary embodiment.
FIG. 4 is a graph showing the viability of natural killer cells according to an exemplary embodiment.

### Modes of the Invention

Hereinafter, the present invention will be described in more detail through Examples. However, these Examples are provided only for exemplarily describing the present invention, and the scope of the present invention is not limited by these Examples.

### Example 1. Manufacture of natural killer cells in which cytokine is linked to linker

To manufacture natural killer cells in which a cytokine is linked to a linker, a vector including a transmembrane domain, a linker, and a polynucleotide encoding the cytokine was constructed.

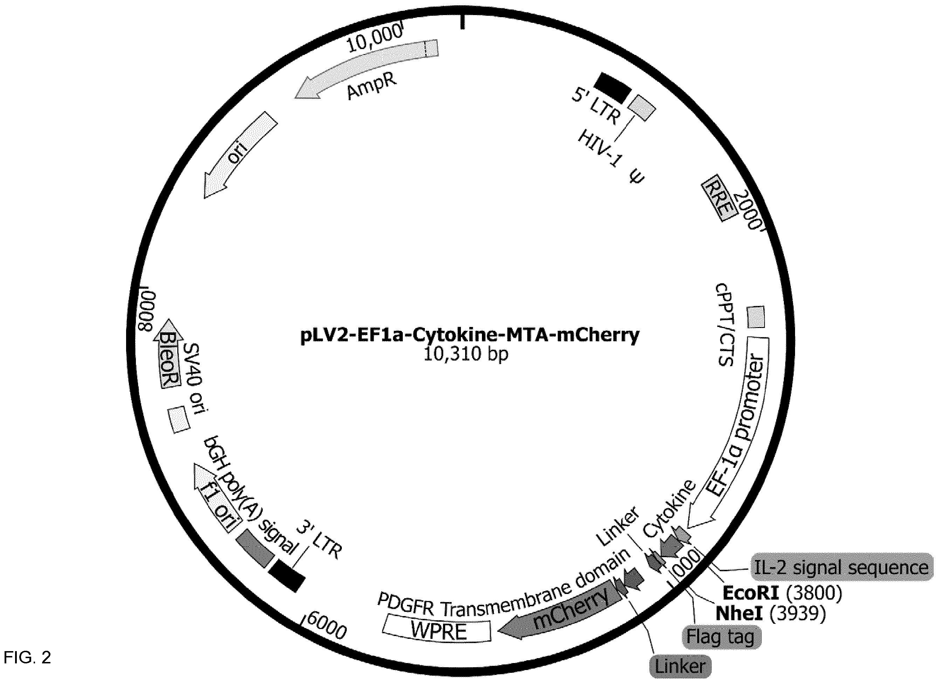

Specifically, PCR primers were designed such that each cDNA could be introduced into a pair of Sfil restriction enzyme recognition sites compatible with each end of a PCR amplified insert in a lentiviral vector pLV2-EF1a-MTA. After an Sfil recognition region was digested, a lentiviral cytokine plasmid for each cytokine was constructed by individually ligating the insert into the Sfil-digested lentiviral vector. The insert was designed so as to include a structure of [cytokine-flexible linker-transmembrane domain], and a specific structure of the plasmid is as illustrated in FIG. 2.

Next, HEK-293FT cells were co-transfected with the above-manufactured lentiviral plasmid together with pCMVD8.9 and pVSVg viral packaging vectors at a ratio of 1: 1: 1. After the cells were incubated overnight, a DNA lipid complex was removed, and then a fresh medium was added to the cells. 48 hours later, an upper layer liquid containing the virus was collected, and the collected supernatant was filtered using a 0.22-µm polyether sulfone membrane filter unit (Millipore). The lentiviral particles obtained from the procedure were added to NK-92 cells (KCTC, Korean Collection for Type Cultures) in a growth medium containing 8 µg/mL of polybrene, and could be infected using a centrifugal force at a speed of 1200 × g for 90 minutes. After 24 hours under the conditions of 37°C and 5% CO₂, the cells were incubated for 2 days or more after replacing the medium with a new medium. The medium was purchased from Thermo Fisher Scientific (Waltham, USA) and used.

The types of cytokines manufactured by the above method are shown in the following Table 1.

**[Table 1]**

| **NO** | **Antibiotic Resistance** | **Vector Name** | **Sequence Primer** | **Entrez Gene List** | **Gene Symbol** | **NCBI. GeneID List** | **NCBI. GeneSymbol List** |
|---|---|---|---|---|---|---|---|
| 1 | Amp | pCR4-TOPO | M13 (-21), M13 reverse, T7, T3 | 57152 | SLURP 1 [Homo sapiens] | 57152 | SLURP 1 |
| 2 | Kan | pCR-BluntII-TOPO | M13(-21), M13 reverse, T7, sp6 | 651 | BMP3 [Homo sapiens] | 651 | BMP3 |
| 3 | Kan | pCR-BluntII-TOPO | M13(-21), M13 reverse, T7, sp6 | 3586 | IL10 [Homo sapiens] | 3586 | IL10 |
| 4 | Cam | pDNR-LIB | -21M13 | 3627 | CXCL10 [Homo sapiens] | 3627 | CXCL10 |
| 5 | Cam | pOTB7 | -21M13, M13 reverse, sp6, T7 | 2821 | GPI [Homo sapiens] | 2821 | GPI |
| 6 | Cam | pDNR-LIB | -21M13 | 6356 | CCL11 [Homo sapiens] | 6356 | CCL11 |
| 7 | Amp | pCR4-TOPO | M13 (-21), M13 reverse, T7, T3 | 9966 | TNFSF15 [Homo sapiens] | 9966 | TNFSF15 |
| 8 | Amp | pCR4-TOPO | M13 (-21), M13 reverse, T7, T3 | 6346 | CCL1 [Homo sapiens] | 6346 | CCL1 |
| 9 | Amp | pPCR-Script Amp SK(+) | M13(-21), M13 reverse, T7, T3 | 56477 | CCL28 [Homo sapiens] | 56477 | CCL28 |
| 10 | Amp | pDNR-Dual | M13(-21), T7 | 3558 | IL2 [Homo sapiens] | 3558 | IL2 |
| 11 | Amp | pCMV-SPORT6 | sp6, T7, - 21M13, M13 reverse | 6358 | CCL14 [Homo sapiens] | 6358 | CCL14 |
| 12 | Amp | pINCY | -21M13, M13 reverse, T7, sp6 | 3589 | IL11 [Homo sapiens] | 3589 | IL11 |
| 13 | Amp | pCMV-SPORT6 | sp6, T7, - 21M13, M13 reverse | 6351 | CCL4 [Homo sapiens] | 6351 | CCL4 |
| 14 | Amp | pCMV-SPORT6 | sp6, T7, - 21M13, M13 reverse | 6347 | CCL2 [Homo sapiens] | 6347 | CCL2 |
| 15 | Amp | pCMV-SPORT6 | sp6, T7, - 21M13, M13 reverse | 6367 | CCL22 [Homo sapiens] | 6367 | CCL22 |
| 16 | Amp | pCMV-SPORT6 | sp6, T7, - 21M13, M13 reverse | 6352 | CCL5 [Homo sapiens] | 6352 | CCL5 |
| 17 | Amp | pDrive | T7, sp6 | 6368 | CCL23 [Homo sapiens] | 6368 | CCL23 |

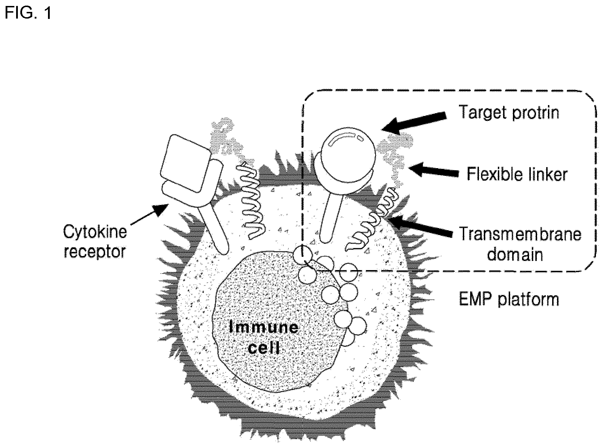

As a result, as illustrated in FIG. 1, natural killer cells were manufactured in which a cytokine was linked to a plasma membrane of the natural killer cells via a linker by a transmembrane domain. The proliferation and activation of cells may be induced by binding the cytokine to a cytokine receptor present on the surface of natural killer cells to continuously stimulate the natural killer cells.

### Experimental Example 1. Analysis of proliferative ability and viability of natural killer cells linked with cytokine by linker

In order to analyze the proliferative ability and viability of natural killer cells designed to be linked with the cytokine IL-2 of Example 1 by a linker, the proportion of living cells after being cultured for a certain period of time without supplying the cytokine was confirmed.

Specifically, natural killer cells in which interlukin-2 was linked to the plasma membrane via a linker through lentiviral infection were isolated using a flow cytometry device, and these cells were cultured in a medium obtained by adding 0.2 mM myo-inositol, 0.1 mM 2-mercarptoethanol, 0.02 mM folic acid, 12.5% horse serum, and 12.5% FBS to an MEM alpha medium and including 100 U/mL interleukin-2 for a week. Thereafter, NK-92 cells to which the cytokine was not attached and NK-92 cells to which interleukin-2 was attached were used as a control and an experimental group, respectively, and cultured for a certain period of time after changing the medium to a medium in which only interleukin-2 was excluded from the same medium composition, and then the number of living cells was confirmed by staining with Trypan blue 1:1. The medium was purchased from Thermo Fisher Scientific (Waltham, USA) and materials to be added were purchased from Sigma Aldrich (St. Louis, USA) and BD (Franklin Lakes, USA), and used.

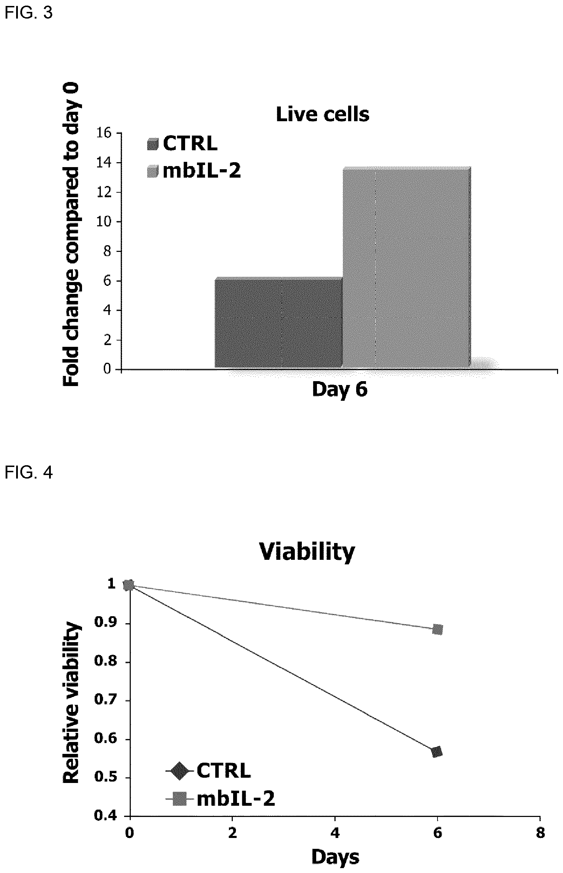

The proliferative ability and viability of natural killer cells were analyzed by calculating cells stained blue with Trypan blue as dead cells and calculating unstained cells as living cells, and the results are illustrated in FIGS. 3 and 4, respectively.

FIG. 3 is a graph showing the proliferative ability of natural killer cells according to an exemplary embodiment.

FIG. 4 is a graph showing the viability of natural killer cells according to an exemplary embodiment.

As illustrated in FIGS. 3 and 4, it was confirmed that the natural killer cells according to a specific example increased their proliferation by 2-fold or more in an environment where external cytokines were absent compared to the natural killer cells of the control which was not genetically manipulated, and it was also confirmed that the cell viability also increased by about 1.6-fold or more.

These results mean that the cytokine linked to the surface of the natural killer cells according to a specific example is proliferated and activated without a separate supply of cytokine by stimulating itself without affecting other cells.

## Claims

1. Genetically engineered immune cells comprising: a transmembrane domain; a cytokine; and a peptide linker for linking the cytokine to the transmembrane domain.

2. The immune cells of claim 1, wherein the transmembrane domain is a transmembrane domain of receptor tyrosine kinases (RTKs).

3. The immune cells of claim 2, wherein the transmembrane domain of receptor tyrosine kinases may be a transmembrane domain of any one receptor selected from the group consisting of an epidermal growth factor receptor, an insulin receptor, a platelet-derived growth factor receptor, a vascular endothelial growth factor receptor, a fibroblast growth factor receptor, a cholecystokinin (CCK) receptor, a neurotrophic factor (NGF) receptor, a hepatocyte growth factor (HGF) receptor, an ephrin (Eph) receptor, an angiopoietin receptor, and a related to receptor tyrosine kinase (RTK) receptor.

4. The immune cells of claim 1, wherein the cytokine is any one selected from the group consisting of the bone morphogenetic protein (BMP) family, the chemokine ligand (CCL) family, the CKLF-like MARVEL transmembrane domain-containing member (CMTM) family, the C-X-C motif ligand (CXCL) family, the growth/differentiation factor (GDF) family, growth hormones, the interferon (IFN) family, the interleukin (IL) family, the tumor necrosis factor superfamily (TNFSF), glycophosphatidylinositol (GPI), secreted *Ly-6*/*uPAR*-related protein 1 (SLUPR-1), secreted *Ly-6*/*uPAR*-related protein 2 (SLUPR-2) and a combination thereof.

5. The immune cells of claim 4, wherein the interleukin is IL1, IL2, IL3, IL4, IL5, IL6, IL7, IL8 (CXCL8), IL9, IL10, IL11, IL12, IL13, IL14, IL15, IL16, IL17, IL18, IL19, IL20, IL21, IL22, IL23, IL24, IL25, IL26, IL27, IL28, IL29, IL30, IL31, IL32, IL33, IL35, or IL36.

6. The immune cells of claim 4, wherein the IFN family is IFN-α, IFN-β, IFN-γ, IFN-ε, IFN-κ or IFN-ω.

7. The immune cells of claim 4, wherein the TNFSF is TNF, CD40L (TNFSF5), CD70 (TNFSF7; CD27L), EDA, FASL (TNFSF6), LTA (TNFSF1), LTB (TNFSF3), TNFSF4 (OX40L), TNFSF8 (CD153), TNFSF9 (4-1BBL), TNFSF10 (TRAIL), TNFSF11 (RANKL), TNFSF12 (TWEAK), TNFSF13, TNFSF13B, TNFSF14, TNFSF15, or TNFSF18.

8. The immune cells of claim 1, wherein the peptide linker is a flexible linker, and comprises 1 to 400 amino acid residues.

9. The immune cells of claim 1, wherein the peptide linker is (GGGGS)ₙ (SEQ ID NO: 1), (SGGGG)ₙ (SEQ ID NO: 2), (SRSSG)ₙ (SEQ ID NO: 3), (SGSSC)ₙ (SEQ ID NO: 4), (GKSSGSGSESKS)ₙ (SEQ ID NO: 5), (RPPPPC)ₙ (SEQ ID NO: 6), (SSPPPPC)ₙ (SEQ ID NO: 7), (GSTSGSGKSSEGKG)ₙ (SEQ ID NO: 8), (GSTSGSGKSSEGSGSTKG)ₙ (SEQ ID NO: 9), (GSTSGSGKPGSGEGSTKG)ₙ (SEQ ID NO: 10), or (EGKSSGSGSESKEF)ₙ (SEQ ID NO: 11), and n is an integer from 1 to 20.

10. The immune cells of claim 1, wherein the immune cells are transformed with a vector comprising a polynucleotide encoding a fusion protein comprising: a transmembrane domain; a cytokine; and a peptide linker for linking the cytokine to the transmembrane domain.

11. The immune cells of claim 10, wherein the vector is selected from the group consisting of a lentivirus, adenovirus, adeno-associated virus, retrovirus, herpes simplex virus, and vaccinia virus.

12. The immune cells of claim 1, wherein the cytokine binds to a cytokine receptor present on the surface of the immune cell to continuously stimulate the cell.

13. The immune cells of claim 1, wherein the immune cells are any one selected from the group consisting of macrophages, B lymphocytes, T lymphocytes, mast cells, monocytes, dendritic cells, eosinophils, natural killer cells, basophils, and neutrophils.

14. The immune cells of claim 1, wherein the immune cells are NK-92 cells.

15. A cell therapeutic agent comprising the immune cells of claim 1 or a cell population thereof as an active ingredient.

16. A pharmaceutical composition for preventing or treating cancer, comprising the immune cells of claim 1, or a cell population thereof as an active ingredient.

17. A pharmaceutical composition for preventing or treating an infectious disease, comprising the immune cells of claim 1, or a cell population thereof as an active ingredient.
